# EUROPÄISCHE PATENTSCHRIFT

(11) **EP 1 094 853 B1**
(45) Veröffentlichungstag und Bekanntmachung des Hinweises auf die Patenterteilung: **30.06.2004**
(21) Anmeldenummer: 99920636.0
(22) Anmeldetag: 10.04.1999
(51) Int. Cl.: A61M 1/06

(54) **MILCH-ABSAUGVORRICHTUNG**
DEVICE FOR ABSTRACTING MILK
DISPOSITIF D'ASPIRATION DE LAIT

(30) Priorität: 16.04.1998 DE 19816776
(43) Veröffentlichungstag der Anmeldung: 02.05.2001
(73) Patentinhaber: Kaweco GmbH, 71254 Ditzingen (DE)
(72) Erfinder: KIRCHNER, Hansjörg, D-71706 Markgröningen (DE)
(74) Vertreter: Fleck, Hermann-Josef, Dr.-Ing.
(86) Internationale Anmeldenummer: PCT/EP1999/002437
(87) Internationale Veröffentlichungsnummer: WO 1999/053976

(56) Entgegenhaltungen:
- EP-A- 0 116 186
- EP-A- 0 237 474
- EP-A- 0 727 234
- DE-U- 8 812 030
- US-A- 5 049 126
- US-A- 5 542 921

## Beschreibung

Die Erfindung bezieht sich auf eine Milch-Absaugvorrichtung mit einem an einem Aufnahmebehältnis anschließbaren Absaugtubus, der zum Ansetzen an der Mutterbrust einen sich nach außen trichterförmig öffnenden Ansatzteil mit einem auf dessen Außenrand aufgesetzten und von außen nach innen von der Innenseite des Ansatzteils zunehmend beabstandeten trichterförmigen, elastischen Einsatz mit einer elastischen Membran aufweist, welcher mit einem zum Behältnis hin offenen Innenabschnitt in ein zwischen dem Ansatzteil und dem Behältnis angeordnetes röhrenförmiges Übergangsstück ragt.

Eine derartige Milch-Absaugvorrichtung ist in der EP 0 237 474 A1 als bekannt ausgewiesen. Bei dieser bekannten Milch-Absaugvorrichtung ist ein an einem Aufnahmebehältnis für die Milch anschließbarer Absaugtubus vorgesehen, der auf seiner von dem Behältnis abgewandten Seite zum Ansetzen an der Mutterbrust einen nach außen trichterförmig geöffneten Ansatzteil aufweist. In die trichterförmige Öffnung ist ein elastischer, ebenfalls trichterförmiger Einsatz eingesetzt, der einerseits mit einem Stülprand auf dem Außenrand des Ansatzteils gehalten ist und andererseits mit einem fingerhutförmigen Innenabschnitt in ein an dem Ansatzteil des Absaugtubus angeschlossenes, zum Aufnahmebehältnis führendes röhrenförmiges Übergangsstück ragt und darin gehalten ist. Dazu ist der Innenabschnitt über seine Länge mit seiner Außenseite an die Innenseite des Übergangsstücks angrenzend luftdicht eingesetzt und steht mit auf seiner zu dem Aufnahmebehältnis gerichteten Vorderseite über Durchbrüche mit dem Innenraum des Aufnahmebehältnisses in Verbindung, so daß die abgesaugte Milch durch das Übergangsstück in das Behältnis fließen kann. Der trichterförmige Abschnitt des elastischen Einsatzes ist membranartig ausgebildet, wobei ein an den Innenabschnitt angrenzender Wandbereich gegenüber dem übrigen Wandbereich der Membran dünner ausgebildet ist, um einen Bereich erhöhter Elastizität zu erzeugen, der sich beim Ausbilden eines Vakuums innerhalb des Absaugraums an die Mutterbrust in geringem Abstand von der Brustwarze anlegt, wobei in einem zwischen der Innenseite des trichterförmigen Ansatzteils des Absaugtubus und der Außenseite der Membran ein Umgebungsdruck aufrechterhalten wird. Der Umgebungsdruck wird dadurch erzeugt, daß zwischen dem Stülprand des Einsatzes und dem Außenrand des Absaugtubus mittels eines Steges eine Luftdurchlaßöffnung freigelassen wird. Die spezielle Ausbildung der Membran mit unterschiedlicher Wandstärke in verschiedenen Bereichen ist relativ aufwendig, und es kann vorkommen, daß die Verformung der Membran zur Anlage an der Mutterbrust nicht ausreichend ist.

Der Erfindung liegt die Aufgabe zugrunde, eine Milch-Absaugvorrichtung der eingangs angegebenen Art bereitzustellen, die bei einfachem Aufbau eine erhöhte Verformbarkeit des elastischen Einsatzes ergibt.

Diese Aufgabe wird mit den Merkmalen des Anspruchs 1 gelöst. Hiernach ist vorgesehen, daß im Ruhezustand, in dem der Einsatz nicht gegen die Elastizitätskraft ausgelenkt ist, zwischen der Außenseite des Innenabschnitts und der Innenseite des Übergangsstücks ein rundum verlaufender Spalt gebildet ist, daß der Innenabschnitt in dem Übergangsabschnitt in Längsrichtung frei beweglich geführt ist.

Dadurch, daß im Ruhezustand zwischen der Außenseite des sich in das Übergangsstück erstreckenden Innenabschnitts und der Innenseite des Übergangsstücks ein rundum verlaufender Spalt gebildet ist, kann der ebenfalls röhrenförmige Innenabschnitt beim Auslenken gegen die Elastizitätskraft zum Behältnis hin verschoben werden, wobei die Mutterbrust an der Membran bereits im Ruhezustand anliegt und beim Erzeugen eines Vakuums in dem Saugraum eine Saugwirkung an der Mutterbrust entsteht. Dabei ist die Auslenkbarkeit der Membran in axialer Richtung und auch eine gewisse Deformation in Querrichtung leicht und über einen relativ großen Weg möglich, so daß eine gute Anpassung an die Mutterbrust für das Absaugen der Milch bei einem guten Massageeffekt vom großen Durchmesser zum kleinen hin erreicht wird. Zwischen der Innenseite des Ansatzteils und der Außenseite der Membran herrscht auch beim Erzeugen des Vakuums ein ausgeglichener Druck, da der elastische Einsatz an dem Außenrand des Ansatzteils rundum abgedichtet ist.

Ist vorgesehen, daß der Innenabschnitt als im wesentlichen zylinderförmiger Stutzen ausgebildet ist, der an seiner Vorderseite vollständig offen ist und im Ruhezustand des Einsatzes um weniger als die Hälfte seiner Längserstreckung in das Übergangsstück ragt, daß sich das Übergangsstück nach vorne zum Behältnis hin verjüngt und daß der Innenabschnitt mit seinem vorderen umlaufenden äußeren Rand nach Verschiebung nach vorn mit der Innenseite des Übergangsstücks in Kontakt tritt, bevor sich der elastische Einsatz mit seiner Außenseite an die Innenseite des trichterförmigen Ansatzteils anlegt, so wird die Vorwärtsverschiebung des Innenabschnitts und damit auch der Membran in der Endphase gedämpft. Auch bei einer Verkippung des Innenabschnitts gegenüber der axialen Richtung wird der Druck in dem Raum zwischen der Außenseite der Membran und der Innenseite des Ansatzteils dadurch sichergestellt, daß in der Außenseite des Innenabschnitts mindestens eine längsverlaufende Rille vorgesehen ist.

Eine für die Herstellung und die Wirkungsweise günstige Ausbildung des elastischen Einsatzes besteht darin, daß der an dem Innenabschnitt nach außen hin anschließende Abschnitt des Einsatzes eine kegelstumpfförmige elastische Membran ist.

Ein einfaches, abgedichtetes Aufsetzen des elastischen Einsatzes auf den Außenrand des Ansatzteils bei guter Abdichtung nach außen wird dadurch erzielt, daß die elastische Membran auf ihrer von dem Innenabschnitt abgekehrten Außenseite einen ununterbrochen umlaufenden, an dem Außenrand des Ansatzteils abnehmbar festgelegten elastischen Stülprand aufweist.

Das Ansetzen der Absaugvorrichtung an der Mutterbrust und die Vorgänge beim Absaugen der Milch können ungehindert beobachtet werden, wenn vorgesehen ist, daß der Absaugtubus und der Einsatz aus vollkommen klarem, durchsichtigem Material hergestellt sind.

Für eine einfache Herstellung ist vorteilhaft, daß der elastische Einsatz in seinem trichterförmigen Bereich eine gleichbleibende Wandstärke aufweist.

Der Massageeffekt kann durch die Maßnahme unterstützt werden, daß der aus hartem Material bestehende Ansatzteil zumindest abschnittsweise nach innen oder außen gewölbt ist.

Die Erfindung wird nachfolgend anhand eines Ausführungsbeispiels unter Bezugnahme auf die Zeichnungen näher erläutert. Es zeigen:
- Fig. 1A bis 1C: einen Einsatz eines Absaugtubus für eine Milch-Absaugvorrichtung von der Öffnungsseite aus, im Querschnitt bzw. ein Detail und
- Fig. 2A bis 2B: einen Absaugtubus im Querschnitt und in teilweise geschnittener Draufsicht.

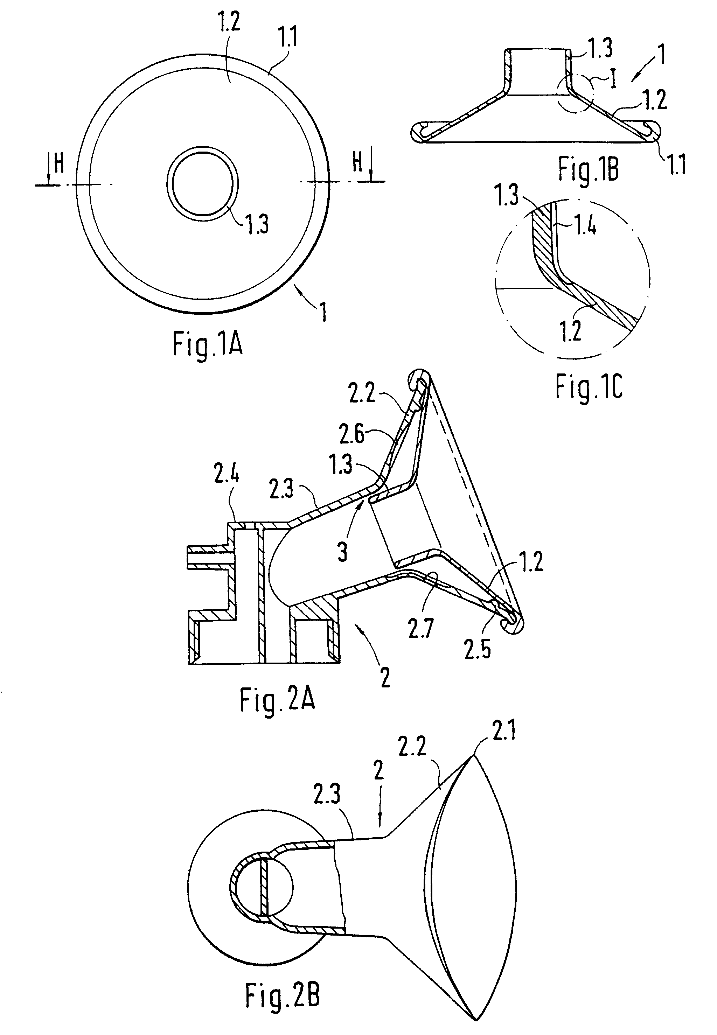

In Fig. 2A ist ein Absaugtubus 2 einer Milch-Absaugvorrichtung im Querschnitt gezeigt, der mit einem Aufsatzabschnitt 2.4 auf ein (nicht gezeigtes) Aufnahmebehältnis aufgesetzt und an eine (ebenfalls nicht gezeigte) Pumpvorrichtung angeschlossen wird. Der Aufsatzabschnitt 2.4 geht über ein schräg nach oben verlaufendes, im wesentlichen röhrenförmiges Übergangsstück 2.3 in einen sich nach außen hin öffnenden trichterförmigen Ansatzteil 2.2 mit einem umlaufenden Außenrand 2.1 über. Auf der Innenseite des Ansatzteiles 2.2 sind für eine zusätzliche Massagewirkung ein oder mehrere Mulden 2.6 angeformt. Vorliegend ist das Übergangsstück 2.3 sich nach außen hin leicht konisch vergrößernd ausgebildet. In Fig. 2B ist der Absaugtubus 2 in Draufsicht, teilweise geschnitten dargestellt.

In Fig. 2A ist gestrichelt ein in den aus relativ hartem Material bestehender Absaugtubus 2 eingesetzter Einsatz 1 wiedergegeben, der mit einem Stülprand 1.1 auf dem Außenrand 2.1 des trichterförmigen Ansatzteils 2.2 abnehmbar und gedichtet festgelegt ist. Zum zusätzlichen Abdichten kann auf der Innenseite des trichterförmigen Ansatzteiles 2.2 auch eine zusätzliche Dichtwulst 2.5 in der Nähe des Außenrandes 2.1 vorgesehen sein. Der gummielastische, aus hygienischem Kunststoff bestehende Einsatz erstreckt sich mit einer ebenfalls trichterförmigen Membran 1.2 in den trichterförmigen Ansatzteil 2.2, wobei zwischen der Innenseite des Ansatzteils 2.2 und der Außenseite der Membran 1.2 ein sich zunehmend vergrößernder umlaufender Abstand gebildet ist. Die Membran 1.2 geht auf ihrer zu dem Aufnahmebehältnis gerichteten Randseite in einen als röhrenförmiger Stutzen ausgebildeten Innenabschnitt 1.3 über, der sich geringfügig, z.B. weniger als die Hälfte seiner Längsausdehnung konzentrisch in das Übergangsstück 2.3 erstreckt, wobei zwischen dem Außenumfang des Innenabschnitts 1.3 und dem Innenumfang des Übergangsstücks 2.3 zumindest im Ruhezustand ein geringer Spalt freigelassen ist, über den der Innenraum des Übergangsstücks 2.3 mit dem zwischen der Innenseite des Ansatzteils 2.2 und der Außenseite der Membran 1.2 gebildeten Raum in Verbindung steht, so daß zwischen diesen Räumen auch beim Erzeugen eines Vakuums ein ausgeglichener Druck herrscht.

In den Fig. 1A bis 1C ist der Einsatz 1 im einzelnen dargestellt, wobei die Fig. 1A eine Draufsicht von der Öffnungsseite aus, die Fig. 1B einen Schnitt längs der in Fig. 1A angegebenen Schnittlinie H-H und Fig. 1C ein Detail I gemäß Fig. 1B zeigt. Wie aus den Fig. 1B und 1C ersichtlich, ist die Wandstärke des Innenabschnitts 1.3 größer als die Wandstärke der Membran 1.2, die somit eine erhöhte Elastizität besitzt. In der Außenseite des Innenabschnitts 1.3 ist mindestens eine Rille 1.4 oder Nut eingeformt, die einen Druckausgleich zwischen dem Innenraum des Übergangsstücks 2.3 und dem Raum zwischen der Innenseite des Ansatzteils 2.2 und der Außenseite der Membran 1.2 auch dann sicherstellt, wenn der röhrenförmige Innenabschnitt 1.3 beim Ansetzen an die Mutterbrust gegen die Längsachse verkippt wird. Alternativ oder zusätzlich kann eine Rille 2.7 auch an der Innenseite des Ansatzteils 3 und des Übergangsstückes 2.3 vorgesehen sein.

Beim Ansetzen an die Mutterbrust liegt die Brustwarze im wesentlichen auf oder in der Nähe der Achse des Übergangsstücks 2.3 und des Innenabschnitts 1.3. Die Membran läßt sich in axialer Richtung gegen die Elastizitätskräfte leicht nach innen verschieben, so daß sie sich beim Ansetzen dichtend an die Mutterbrust anlegen kann und ein nach außen abgedichteter Saugraum mit dem Innenraum des Übergangsstücks 2.3 und den übrigen Hohlräumen zum Aufnahmebehältnis ergibt, in dem sich mittels einer Saugpumpe das zum Abpumpen der Milch erforderliche Vakuum erzeugen läßt. Die Membran 1.2 kann beim Anlegen an die Mutterbrust leicht nach vorne in Richtung auf das Aufnahmebehältnis verschoben werden, wobei der Innenabschnitt 1.3 weiter in das Übergangsstück 2.3 eingeschoben wird, und zwar maximal so weit, daß sich die Membran 1.2 an die Innenseite des Ansatzteils 2.2 anlegt. Die Ausbildung kann derart sein, daß der vordere äußere Rand des Innenabschnitts 1.3 mit der Innenseite des leicht konischen Übergangsstücks 2.3 in Kontakt tritt, wodurch das weitere Einschieben des Innenabschnitts 1.3 in das Übergangsstück 2.3 gedämpft wird und die Membran 1.2 nicht abrupt gegen die Innenseite des Ansatzteils 2.2 in Anlage kommt. Dadurch können das Ansetzen der Absaugvorrichtung an die Mutterbrust und die Absaugvorgänge begünstigt werden.

Zum Beobachten der Absaugvorgänge und des Fließens der Milch sind der Absaugtubus 2 und der Einsatz 1 vorzugsweise aus klarem, durchsichtigem Kunststoff hergestellt.

## Patentansprüche

1. Milch-Absaugvorrichtung mit einem an einem Aufnahmebehältnis anschließbaren Absaugtubus (2), der zum Ansetzen an der Mutterbrust einen sich nach außen trichterförmig öffnenden Ansatzteil (2.2) sowie einen auf dessen Außenrand aufgesetzten und von außen nach innen von der Innenseite des Ansatzteils (2.2) umlaufend zunehmend beabstandeten trichterförmigen, elastischen Einsatz (1) mit einer elastischen Membran (1.2) aufweist, welcher mit einem zum Behältnis hin offenen Innenabschnitt (1.3) in ein zwischen dem Ansatzteil (2.2) und dem Behältnis angeordnetes röhrenförmiges Übergangsstück (2.3) ragt, worin der elastische Einsatz (1) an dem Außenrand des Ansatzteils (2.2) rundum abgedichtet ist.
**dadurch gekennzeichnet,**
**dass** im Ruhezustand, in dem der Einsatz (1) nicht gegen die Elastizitätskraft ausgelenkt ist, zwischen der Außenseite des Innenabschnitts (1.3) und der Innenseite des Übergangsstücks (2.3) ein rundum verlaufender Spalt gebildet ist,
**dass** der Innenabschnitt in dem Übergangsabschnitt (2.3) in Längsrichtung frei beweglich geführt ist.

2. Absaugvorrichtung nach Anspruch 1,
**dadurch gekennzeichnet,**
**dass** der Innenabschnitt (1.3) als im Wesentlichen zylinderförmiger Stutzen ausgebildet ist, der an seiner Vorderseite vollständig offen ist und im Ruhezustand des Einsatzes (1) um weniger als die Hälfte seiner Längserstreckung in das Übergangsstück (2.3) ragt,
**dass** sich das Übergangsstück (2.3) nach vorne zum Behältnis hin verjüngt und
**dass** der Innenabschnitt (1.3) mit seinem vorderen umlaufenden äußeren Rand nach Verschiebung nach vorn mit der Innenseite des Übergangsstückes (2.3) in Kontakt tritt, bevor sich die elastische Membran (1.2) mit ihrer Außenseite an die Innenseite des trichterförmigen Ansatzteils (2.2) anlegt.

3. Absaugvorrichtung nach Anspruch 1 oder 2,
**dadurch gekennzeichnet,**
**dass** in der Außenseite des Innenabschnitts (1.3) oder in der Innenseite des diesen umgebenden Übergangsstückes (2.3) mindestens eine längs verlaufehde Rille (1.4) vorgesehen ist.

4. Absaugvorrichtung nach einem der vorhergehenden Ansprüche,
**dadurch gekennzeichnet,**
**dass** der an dem Innenabschnitt (1.3) nach außen hin anschließende Abschnitt des Einsatzes (1) eine kegelstumpfförmige elastische Membran (1.2) ist.

5. Absaugvorrichtung nach einem der vorhergehenden Ansprüche,
**dadurch gekennzeichnet,**
**dass** die elastische Membran (1.2) auf ihrer von dem Innenabschnitt (1.3) abgekehrten Außenseite einen ununterbrochen umlaufenden, an dem Außenrand des Ansatzteils (2.2) abnehmbar festgelegten elastischen Stülprand (1.1) aufweist.

6. Absaugvorrichtung nach einem der vorhergehenden Ansprüche,
**dadurch gekennzeichnet,**
**dass** der Absaugtubus (2) und der Einsatz (1) aus vollkommen klarem, durchsichtigem Material hergestellt sind.

7. Absaugvorrichtung nach einem der vorhergehenden Ansprüche,
**dadurch gekennzeichnet,**
**dass** der elastische Einsatz (1) in seinem trichterförmigen Bereich eine gleich bleibende Wandstärke aufweist.

8. Absaugvorrichtung nach einem der vorhergehenden Ansprüche,
**dadurch gekennzeichnet,**
**dass** der aus hartem Material bestehende Ansatzteil (2.2) zumindest abschnittsweise nach innen oder außen gewölbt ist.

## Claims

1. Device for extracting milk having an extracting tube (2), which is connectable to a receiving container and which, for placing on the mother's breast, has an attachment piece (2.2), which opens outwards in a funnel-shaped manner, as well as a funnel-shaped, resilient insert (1), which is mounted on the outer edge of the attachment piece and has a circular spacing which becomes greater between the outside and the inside of the attachment piece (2.2), said insert (1) having a resilient membrane (1.2), which projects with an inner portion (1.3), which is open towards the container, into a tubular transition piece (2.3), which is disposed between the attachment piece (2.2) and the container, wherein the resilient insert (1) is sealed all the way round on the outer edge of the attachment piece (2.2), **characterised in that** in the non-operative condition, in which the insert (1) is not guided in opposition to the force of elasticity, a gap is formed extending right around between the outside of the inner portion (1.3) and the inside of the transition piece (2.3), **in that** the inner portion is guided in the transition portion (2.3) so as to be freely displaceable in the longitudinal direction.

2. Device for extracting milk according to claim 1, **characterised in that** the inner portion (1.3) is in the form of a substantially cylindrical support piece, which is completely open at its front end and in the non-operative condition of the insert (1) protrudes by less than half of its longitudinal extension into the transition piece (2.3), **in that** the transition piece (2.3) tapers forwards towards the container, and **in that** the inner portion (1.3), after it has been pushed forwards, comes into contact with the inside of the transition portion (2.3) with its front circumferential outer edge, before the resilient membrane (1.2) abuts against the inside of the funnel-shaped attachment piece (2.2) with its outside.

3. Device for extracting milk according to claim 1 or 2, **characterised in that** at least one longitudinally extending groove (1.4) is provided in the outside of the inner portion (1.3) or in the inside of the transition portion (2.3) surrounding said inner portion.

4. Device for extracting milk according to one of the preceding claims, **characterised in that** the portion of the insert (1), which connects outwardly to the inner portion (1.3), is a truncated cone-shaped resilient membrane (1.2).

5. Device for extracting milk according to one of the preceding claims, **characterised in that** the resilient membrane (1.2) has on its outside, which is remote from the inner portion (1.3), an uninterruptedly circumferential, resilient U-shaped edge (1.1.), which is secured to the outer edge of the attachment part (2.2) so as to be detachable.

6. Device for extracting milk according to one of the preceding claims, **characterised in that** the extracting tube (2) and the insert (1) are produced from completely clear, transparent material.

7. Device for extracting milk according to one of the preceding claims, **characterised in that** the thickness of the wall of the resilient insert (1) remains constant in its funnel-shaped region.

8. Device for extracting milk according to one of the preceding claims, **characterised in that** the attachment part (2.2), which consists of hard material, is curved inwards or outwards at least in portions.

## Revendications

1. Dispositif tire-lait comprenant un tube d'aspiration (2), qui est destiné à être raccordé à un contenant collecteur et qui comporte un élément de pose (2.2), s'ouvrant vers l'extérieur en forme d'entonnoir et destiné à être posé contre le sein maternel, ainsi qu'un insert (1) souple, destiné à être posé sur le bord extérieur dudit élément de pose et dont le pourtour en forme d'entonnoir s'écarte progressivement, de l'extérieur vers l'intérieur, de la paroi intérieure de l'élément de pose (2.2), lequel insert est muni d'une membrane élastique (1.2) et, avec une partie intérieure (1.3) ouverte vers le contenant, s'engage dans une pièce de transition (2.3) tubulaire, qui est agencée entre l'élément de pose (2.2) et le contenant et dans laquelle l'insert (1) souple est rendu étanche sur tout le pourtour au niveau du bord extérieur de l'élément de pose (2.2), **caractérisé en ce que**, en position de repos, dans laquelle l'insert (1) n'est pas déplacé à l'encontre de la force élastique, une fente périphérique est formée entre la face extérieure de la partie intérieure (1.3) et la face intérieure de la pièce de transition (2.3), **en ce que** la partie intérieure est logée de manière librement mobile dans le sens longitudinal dans la partie de transition (2.3).

2. Dispositif tire-lait selon la revendication 1, **caractérisé en ce que** la partie intérieure (1.3) est conçue sous forme de manchon sensiblement cylindrique, qui est entièrement ouvert sur le côté avant et qui, dans la position de repos de l'insert (1), pénètre dans la pièce de transition (2.3) sur une distance inférieure à moins de la moitié de sa dimension longitudinale, **en ce que** la partie de transition (2.3) se rétrécit vers l'avant en direction du contenant, et **en ce que** la partie intérieure (1.3) avec son bord extérieur avant périphérique, après un déplacement vers l'avant, entre en contact avec la face intérieure de la pièce de transition (2.3) avant que la membrane (1.2) élastique vienne en appui avec sa face extérieure contre la face intérieure de l'élément de pose (2.2) en forme d'entonnoir.

3. Dispositif tire-lait selon la revendication 1 ou 2, **caractérisé en ce qu'**il est prévu au moins une cannelure (1.4) s'étendant longitudinalement dans la face extérieure de la partie intérieure (1.3) ou dans la face intérieure de la pièce de transition (2.3) entourant celle-ci.

4. Dispositif tire-lait selon l'une quelconque des revendications précédentes, **caractérisé en ce que** la partie de l'insert (1) prolongeant vers l'extérieur la partie intérieure (1.3) est une membrane (1.2) élastique en forme de cône tronqué.

5. Dispositif tire-lait selon l'une quelconque des revendications précédentes, **caractérisé en ce que** la membrane (1.2) élastique, sur sa face extérieure détournée de la partie intérieure (1.3), comporte un bord de recouvrement (1.1) élastique périphérique en continu et fixé de manière amovible contre le bord extérieur de l'élément de pose (2.2).

6. Dispositif tire-lait selon l'une quelconque des revendications précédentes, **caractérisé en ce que** le tube d'aspiration (2) et l'insert (1) sont réalisés dans un matériau transparent entièrement limpide.

7. Dispositif tire-lait selon l'une quelconque des revendications précédentes, **caractérisé en ce que** l'insert (1) souple comporte une épaisseur de paroi uniforme dans sa partie en forme d'entonnoir.

8. Dispositif tire-lait selon l'une quelconque des revendications précédentes, **caractérisé en ce que** l'élément de pose (2.2), réalisé en matériau dur, est cintré au moins par sections vers l'intérieur ou vers l'extérieur.
